# EUROPEAN PATENT SPECIFICATION

(11) **EP 0 633 262 B1**
(45) Date of publication and mention of the grant of the patent: **17.01.2001**
(21) Application number: 94610036.9
(22) Date of filing: 29.06.1994
(51) Int. Cl.: C07D 471/04, A61K 31/40, A61K 31/435

(54) **Novel isatineoxime derivatives, their preparation and use**
Isatinoxim-Derivate, ihre Herstellung und Verwendung
Dérivés d'isatineoxime, leur préparation et leur application

(30) Priority: 07.07.1993 DK 81593
(43) Date of publication of application: 11.01.1995
(73) Proprietor: NEUROSEARCH A/S, 2750 Ballerup (DK)
(72) Inventor: Wätjen, Frank, DK-2730 Herlev (DK); Dahl, Bjarne Hugo, DK-2100 Copenhagen O (DK); Drejer, Jorgen, DK-3500 Vaerlose (DK); Jensen, Leif Helth, DK-1573 Copenhagen V (DK)

(56) References cited:
- EP-A- 0 522 494
- EP-A- 0 529 636
- BIOORGANIC AND MEDICINAL CHEMISTRY LETTERS, vol.3, no.1, 1993 pages 105 - 106 F. WÄTJEN ET AL. 'Isatin oximes - a novel series of bioavailable non-NMDA antagonists'
- EUROPEAN JOURNAL OF PHARMACOLOGY - MOLECULAR PHARMACOLOGY SECTION, vol.246, no.3, 1993 pages 195 - 204 T.H. JOHANSEN ET AL. 'A novel non-NMDA receptor antagonist ...'

## Description

The present invention relates to novel ring fused indole-2,3-dione oxime derivatives, to pharmaceutical compositions comprising these compounds, to the use of these compounds for the manufacture of a medicament, and to a method of preparing the novel compounds of the invention.

### Object of the Invention

It is an object of the present invention to provide novel isatin derivatives which are useful in the treatment of diseases in mammals, including a human, and especially in the treatment of diseases which can be treated by antagonizing an excitatory amino acid of such mammal.

Another object of the present invention is to provide novel pharmaceutical compositions for the treatment of diseases in mammals, including a human, responsive to the blockade of glutamic and aspartic acid receptors.

### Background of the Invention

Excessive excitation by neurotransmitters can cause the degeneration and death of neurons. It is believed that this degeneration is in part mediated by the excitotoxic actions of the excitatory amino acids (EAA) glutamate and aspartate at the N-methyl-D-aspartate (NMDA), the α-amino-3-hydroxy-5-methyl-4-isoxazole propionic acid (AMPA) receptor, and the kainate receptor. This excitotoxic action is responsible for the loss os neurons in cerebrovascular disorders such as cerebral ischemia or cerebral infarction resulting from a range of conditions, such as thromboembolic or haemorrhagic stroke, cerebral vasospasm, hypoglycaemia, cardiac arrest, status epilepticus, perinatal asphyxia, anoxia such as from drowning, pulmonary surgery and cerebral trauma as well as lathyrism, Alzheimer's, and Huntington's diseases.

The compounds of the present invention may also be useful in the treatment of schizophrenia, epilepsy, anxiety, pain and drug addiction.

EP A 529636, published March 3, 1993, and EP A 522494, published January 13, 1993, discloses certain isatineoxime derivatives.

### Summary of the Invention

The invention then, inter alia, comprises the following, alone or in combination:

A compound having the formula 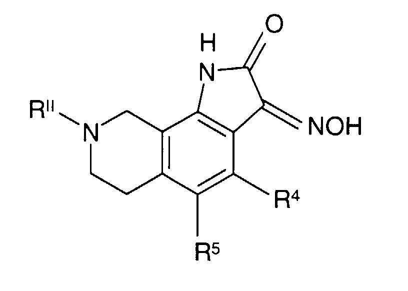 or a pharmaceutically acceptable salt thereof
wherein
R⁴ and R⁵ independently represent hydrogen, halogen, CF₃, CN, NO₂ or a group of the formula SO₂NR¹R² wherein R¹ and R² independently represent hydrogen, C₁-C₆-alkyl which may be straight or branched, or C₃-C₇-cycloalkyl,
and R^{II} represents hydrogen, benzyl, -(C=O)CF₃, C₁₋₆-carboxylic acid acyl, C₁-C₆-alkoxy, C₁-C₆-alkyl which may be straight or branched, or CH₂CO₂R^{VI} wherein R^{VI} represents hydrogen or C₁-C₆-alkyl which may be straight or branched, and
a compound as above wherein R⁴ is hydrogen, R⁵ is hydrogen and R^{II} is methyl , and
a compound as above wherein R⁴ is hydrogen, R⁵ is hydrogen and R^{II} is ethyl , and
a compound as above wherein R⁴ is hydrogen, R⁵ is dimethylsulphamoyl, and R^{II} is methyl , and
a compound as above wherein R⁴ is hydrogen, R⁵ is dimethylsulphamoyl, and R^{II} is ethyl, and
a pharmaceutical composition useful for treatment of a disorder of a mammal which is responsive to the blockage of glutamic and aspartic acid receptors, comprising an amount of a compound as any above which is effective for such purpose together with a pharmaceutically-acceptable carrier, and
the use of a compound as any above, for the manufacture of a medicament useful in the treatment of disorders of a mammal, including a human, responsive to the blockade of glutamic or aspartic receptors, and
the use of a compound as any above for the manufacture of a medicament useful in the treatment of cerebrovascular or psychotic disorders, and
a method of preparing the chemical compound of any of claims 1-5, which method comprises the step of reacting a compound represented by the general formula (II) 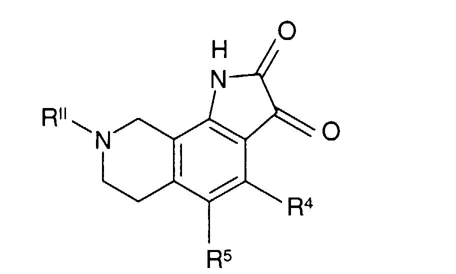 wherein R⁴ R⁵ and R^{II} are as defined in claim 1,
with hydroxylamine or a reactive derivative thereof.

Examples of pharmaceutically-acceptable addition salts include inorganic and organic acid addition salts such as the hydrochloride, hydrobromide, phosphate, sulphate, citrate, lactate, tartrate, maleate, fumarate, mandelate, oxalate, benzoate, ascorbate, cinnamate, benzenesulfonate, methanesulfonate, stearate, succinate, glutamate, salicylate and the acetate. Such salts are formed by procedures well known in the art.

Halogen is fluorine, chlorine, bromine, or iodine.

Alkyl means a straight chained or branched chain of from one to six carbon atoms or cyclic alkyl of from three to seven carbon atoms, including but not limited to, methyl, ethyl, propyl, isopropyl, butyl, isobutyl, t-butyl, pentyl, hexyl, cyclopropyl, cyclobutyl, cyclopentyl, cyclohexyl; methyl, ethyl, propyl and isopropyl are preferred groups.

Cycloalkyl means cycloalkyl of from three to seven carbon atoms.

Alkoxy is O-alkyl, wherein alkyl is as defined above.

The compounds of this invention may exist in unsolvated as well as in solvated forms with pharmaceutically acceptable solvents such as water, ethanol and the like. In general, the solvated forms are considered equivalent to the unsolvated forms for the purposes of this invention.

Some of the compounds of the present invention exist in (+) and (-) forms as well as in racemic forms. Racemic forms can be resolved into the optical antipodes by known methods, for example, by separation of diastereomeric salts thereof, with an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallization of d-or I- (tartrates, mandelates, or camphorsulphonate) salts for example. The compounds of the present invention may also be resolved by the formation of diastereomeric amides by reaction of the compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the compounds of the present invention with an optically active chloroformate or the like.

Additional methods for the resolvation of optical isomers, known to those skilled in the art may be used, and will be apparent to the average skilled in the art. Such methods include those discussed by J. Jaques, A. Collet, and S. Wilen in "Enantiomers, Racemates, and Resolutions", John Wiley and Sons, New York (1981).

Starting materials for the processes described in the present application are known or can be prepared by known processes from commercially available chemicals.

The products of the reactions described herein are isolated by conventional means such as extraction, crystallization, distillation, chromatography, and the like.

### Biological Activity

The compounds of the invention exhibit valuable biological properties because of their strong excitatory amino acid (EAA) antagonizing properties at the AMPA ((RS)-α-amino-3-hydroxy-5-methyl-4-isoxazolepropionic acid) binding site.

The compound 5-dimethylsulfamoyl-8-Methyl-5,7,8,9-tetrahydro-1H-pyrrolo[3,2-h]isoquinoline-2,3-dione-3-oxime for example is active in the AMPA binding assay as described by T. Honoré et al., Neuroscience Letters 54, 27-32 (1985). In the same assay 8-Methyl-5,7,8,9-tetrahydro-1H-pyrrolo[3,2-h]isoquinoline-2,3-dione-3-oxime is also active.

The compounds of the invention exhibit interesting and superior in vivo kinetic properties, for example as measured in the maximum electroshock assay.

### Pharmaceutical Compositions

The compounds of the invention, together with a conventional adjuvant, carrier, or diluent, may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. Tablets containing ten (10) milligrams of active ingredient or, more broadly, one (1) to one hundred (100) milligrams, per tablet, are accordingly suitable representative unit dosage forms.

The compounds of the present invention can be administrated in a wide variety of oral and parenteral dosage forms. It will be obvious to those skilled in the art that the following dosage forms may comprise as the active component, either a compound of the invention or a pharmaceutically acceptable salt of a compound of the invention.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component.

In, tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting vax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting vax, such as a mixture of fatty acid glycerides or cocoa butter, is first melted and the active component is dispersed homogeneously therein, as by stirring. The molten homogenous mixture is then poured into convenient sized molds, allowed to cool, and thereby to solidify.

Liquid form preparations include solutions, suspensions, and emulsions, for example, water or water propylene glycol solutions. For example, parenteral injection liquid preparations can be formulated in solutions in aqueous polyethylene glycol solution.

Aqueous solutions suitable for oral use can be prepared by dissolving the active component in water and adding suitable colorants, flavours, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, and other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilizers, buffers, artificial and natural sweeteners, dispersants, thickeners, solubilizing agents, and the like.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

### Method of Treating

The compounds of this invention are extremely useful in the treatment of central nervous system disorders related to their biological activity. The compounds of this invention may accordingly be administered to a subject, including a human, in need of treatment, alleviation, or elimination of a disorder or disease associated with the biological activity of the compounds. This includes especially excitatory amino acid dependent, including glutamate and/or aspartate dependent psychosis, excitatory amino acid dependent, including glutamate and/or aspartate dependent anoxia, excitatory amino acid dependent, including glutamate and/or aspartate dependent ischemia, excitatory amino acid dependent, including glutamate and/or aspartate dependent Parkinsonism, excitatory amino acid dependent, including glutamate and/or aspartate dependent convulsions and excitatory amino acid dependent, including glutamate and/or aspartate dependent migraine. Suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge.

The following nonlimiting examples illustrate the present invention further.

### Example 1

A solution of potassium nitrate (1.78 g, 8.56 mmol) was added slowly to a solution of 5-bromoisoquinoline in 12 mL H₂SO₄. After stirring for 3 h the reaction mixture was poured onto ice and neutralized with conc. ammonium hydroxide. The yellow precipitate was extracted with ethyl acetate (3x), and the combined organic layers were washed with saturated NaCI, dried over MgSO4, filtered and concentrated. The residue was chromatographed over silica gel (40% ethyl acetate in hexane) to give 5-bromo-8-nitroisoquinoline in 96% yield.

### Example 2

A mixture of 5-bromo-8-nitroisoquinoline (0.99 g, 3.91 mmol) and dimethylsulfate (0.41 mL) in anhydrous DMF (20 mL) was heated at 80°C for 24 h. After removing the DMF in vacuo, the isoquinoline methylammonium salt was obtained (used without further purification).

### Example 3

The isoquinoline salt (3.9 mmol) was dissolved in acetic acid (10 mL) and sodium borohydride (0.15 g, 3.97 mmol) was added. After stirring for 24 h, the reaction mixture was diluted with a mixture of ethyl acetate and water and potassium carbonate was added portionwise to neutralize the acetic acid. The aqueous layer was extracted with ethyl acetate (2x), washed with saturated NaCI, dried over MgSO₄, filtered and evaporated. The residue was chromatographed on silica gel (30% ethyl acetate in hexane) to give the light sensitive N-methyl 5-bromo-8-nitro-1,2,3,4-tetrahydroisoquinoline (0.47 g, 45% yield).

### Example 4

5-bromo-2-methyl-8-nitroisoquinoline (5g) was hydrogenated under standard conditions in ethanol using 5% Pd/C as catalyst. The catalyst was filtered off and the filtrate was evaporated in vacuo. The product was isolated as pale crystals. Yield was 89%, M.p. 148.150°C

### Example 5

### 8-Methyl-5,7,8,9-tetrahydro-1H-pyrrolo[3,2-h]isoquinoline-2,3-dione

3.8 g of the product of example 4, 3.6 g of hydroxylamine hydrochloride, 1.8 ml of chlorale in 70 ml of water and 16 g of disodium sulphate was heated to 60°C for 60 min. The solution was cooled and pH adjusted to 8 with Na₂CO₃. This afforded a crystalline precipitate which was filtered off and washed with water. After drying, the crystals were dissolved in 20 ml of concentrated sulphuric acid and heated with stirring for 60 minutes. The mixture was cooled and crushed ice 100 g was added followed by 15 ml of 30% sodium hydroxide. Thereafter saturated aqueous disodium carbonate was added until pH was 9. The formed precipitate was isolated by filtration. Yield of the title compound was 90%, M.p. 198-200°C.

### Example 6

### 5-dimethylsulfamoyl-8-Methyl-5,7,8,9-tetrahydro-1H-pyrrolo[3,2-h]isoquinoline-2,3-dione-3-oxime

8-Methyl-5,7,8,9-tetrahydro-1H-pyrrolo[3,2-h]isoquinoline-2,3-dione (0.4 g) was dissolved in chlorosulphonic acid (2 ml). The solution was heated to 90°C for 3 min, then cooled and the excess of chlorosulphonic acid was destroyed by addition of neat NaCI, followed by addition of 2 ml water. After some time a precipitate of 3,3-dichloro-8-methyl-5,7,8,9-tetrahydro-1H-pyrrolo[3,2-h]isoquinoline-2-one-5-sulphonylchloride, hydrochloride was formed. The precipitate was filtered off, washed with 4N HCI and dried. Thereafter it was brought into solution in dry THF (20 ml), undissolved inorganic material was removed by filtration. To the THF solution was added dimethylamine until complete reaction (followed by TLC). The reaction mixture was filtered and the solvent was removed by evaporation. This left the crude 3,3-dichloro-5-sulfamoyl-8-methyl-5,7,8,9-tetrahydro-1H-pyrrolo[3,2-h]isoquinoline-2-one as a solid residue (M.p. 270-290°C, decomposition) which was reacted with hydroxylamine, hydrochloride (100 mg) in refluxing methanol (5 ml). After 1 hour reflux the reaction mixture was cooled to ambient temperature and the product (title compound) was filtered off as the monohydrochloride, M.p. (free base) 250-300°C (decomposition).

### Example 7

### 8-Methyl-5,7,8,9-tetrahydro-1H-pyrrolo[3,2-h]isoquinoline-2,3-dione-3-oxime

220 mg of 8-Methyl-5,7,8,9-tetrahydro-1H-pyrrolo[3,2-h]isoquinoline-2,3-dione, 130 mg of disodium carbonate and 83 mg of hydroxylamine hydrochloride in 20 ml of methanol was stirred for 1 hours at reflux temperature. Then water and acetic acid was added to the reaction mixture and sodium hydrogen carbonate was thereafter added until pH ~ 8. The crystalline precipitate was isolated by filtration. Yield of the title compound 120 mg, M.p. 196-197°C.

## Claims

1. A chemical compound represented by the general formula (I) 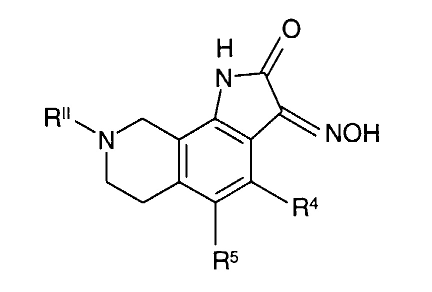 or a pharmaceutically acceptable salt thereof,
wherein,
R⁴ and R⁵ independently represent hydrogen, halogen, CF₃, CN, NO₂ or a group of the formula SO₂NR¹R² wherein R¹ and R² independently represent hydrogen, C₁-C₆-alkyl which may be straight or branched, or C₃-C₇-cycloalkyl,
and R^{II} represents hydrogen, benzyl, -(C=O)CF₃, C₁₋₆-carboxylic acid acyl, C₁-C₆-alkoxy, C₁-C₆-alkyl which may be straight or branched, or CH₂CO₂R^{VI} wherein R^{VI} represents hydrogen or C₁-C₆-alkyl which may be straight or branched.

2. The chemical compound of claim 1, wherein R⁴ and R⁵ represent hydrogen, and R^{II} represents methyl.

3. The chemical compound of claim 1, wherein R⁴ and R⁵ represent hydrogen, and R^{II} represents ethyl.

4. The chemical compound of claim 1, wherein R⁴ represents hydrogen, R⁵ represents dimethylsulphamoyl, and R^{II} represents methyl .

5. The chemical compound of claim 1, wherein R⁴ represents hydrogen, R⁵ represents dimethylsulphamoyl, and R^{II} represents ethyl .

6. A pharmaceutical composition useful for treatment of a disorder of a mammal which is responsive to the blockage of glutamic and aspartic acid receptors, comprising an amount of the chemical compound of any of claims 1-5, which is effective for such purpose, together with a pharmaceutically-acceptable carrier.

7. The use of the chemical compound of any of claims 1-5, for the manufacture of a medicament useful in the treatment of disorders of a mammal, including a human, responsive to the blockade of glutamic or aspartic receptors.

8. The use of the chemical compound of any of claims 1-5, for the manufacture of a medicament useful in the treatment of cerebrovascular or psychotic disorders.

9. A method of preparing the chemical compound of any of claims 1-5, which method comprises the step of reacting a compound represented by the general formula (II) 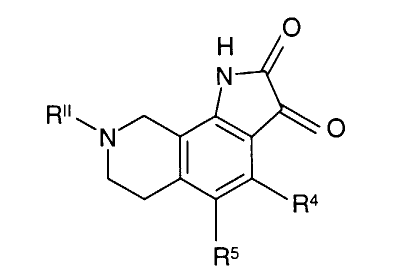 wherein R⁴, R⁵ and R^{II} are as defined in claim 1,
with hydroxylamine or a reactive derivative thereof.

## Patentansprüche

1. Chemische Verbindung wiedergegeben durch die allgemeine Formel (l) 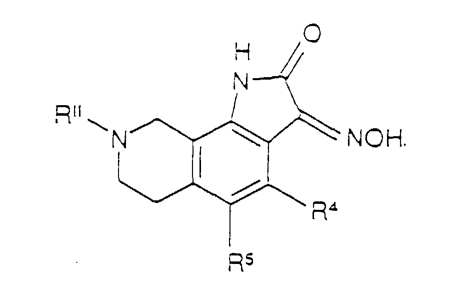 oder ein pharmazeutisch verträgliches Salz davon, worin
R⁴ und R⁵ unabhängig voneinander Wasserstoff, Halogen, CF₃, CN, NO₂ oder eine Gruppe der Formel SO₂NR¹R² bedeuten, worin R¹ und R² unabhängig voneinander Wasserstoff, C₁- bis C₆-Alkyl, das geradkettig oder verzweigt sein kann, oder C₃- bis C₇-Cycloalkyl bedeuten,
und R^{II} Wasserstoff, Benzyl, -(C=O)CF₃, C₁₋₆-Carbonsäureacyl, C₁- bis C₆-Alkoxy, C₁- bis C₆-Alkyl, das geradkettig oder verzweigt sein kann, oder CH₂CO₂R^{VI} bedeutet, worin R^{VI} oder C₁- bis C₆-Alkyl bedeutet, das geradkettig oder verzweigt sein kann.

2. Chemische Verbindung nach Anspruch 1, worin R⁴ und R⁵ Wasserstoff bedeuten und R^{II} Methyl bedeutet.

3. Chemische Verbindung nach Anspruch 1, worin R⁴ und R⁵ Wasserstoff bedeuten und R^{II} Ethyl bedeutet.

4. Chemische Verbindung gemäß Anspruch 1, worin R⁴ Wasserstoff bedeutet, R⁵ Dimethylsulfamoyl bedeutet und R^{II} Methyl bedeutet.

5. Chemische Verbindung nach Anspruch 1, worin R⁴ Wasserstoff bedeutet, R⁵ Dimethylsulfamoyl bedeutet und R" Ethyl bedeutet.

6. Pharmazeutische Zusammnsetzung verwendbar zur Behandlung einer Störung eines Säugers, die auf die Blockade von Glutamin- und Aspartinsäure-Rezeptoren anspricht, enthaltend eine Menge der chemischen Verbindung nach einem der Ansprüche 1-5, die für diesen Zweck wirksam ist, zusammen mit einem pharmazeutisch verträglichen Träger.

7. Verwendung der chemischen Verbindung nach einem der Ansprüche 1-5 zur Herstellung eines Arzneimittels, das bei der Behandlung von Störungen eines Säugers, einschließlich des Menschen, brauchbar ist, die auf die Blockade von Glutamin- oder Asparagin-Rezeptoren ansprechen.

8. Verwendung der chemischen Verbindung nach einem der Ansprüche 1-5 zur Herstellung eines Arzneimittels, das bei der Behandlung von cerebrovaskulären oder psychotischen Störungen brauchbar ist.

9. Verfahren zur Herstellung einer chemischen Verbindung nach einem der Ansprüche 1-5, welches die Stufe der Umsetzung einer durch die allgemeine Formel (II) wiedergegebenen Verbindung 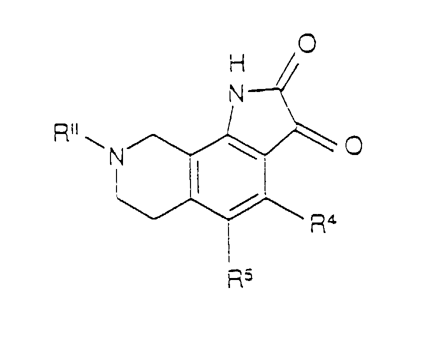 worin R⁴, R⁵ und R^{II} wie in Anspruch 1 definiert sind, mit Hydroxylamin oder einem reaktiven Derivat davon umfaßt.

## Revendications

1. Composé chimique représenté par la formule générale (I) : 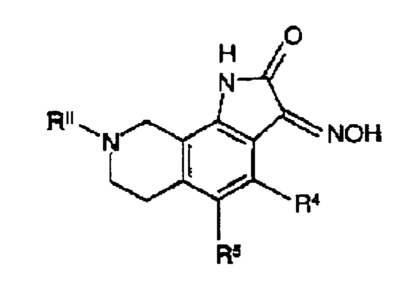 ou un sel pharmaceutiquement acceptable de celui-ci
dans lequel R⁴ et R⁵ représentent indépendamment l'hydrogène, un halogène, CF₃, CN, NO₂ ou un groupe de formule SO₂NR¹R² dans laquelle R¹ et R² représentent indépendamment l'hydrogène, un alkyle en C₁ à C₆ qui peut être linéaire ou ramifié, ou un cycloalkyle en C₃ à C₇,
et R¹¹ représente l'hydrogène, le benzyle, -(C=O)CF₃, un acyle d'acide carboxylique en C_{1 à 6}, un alcoxy en C₁ à C₆, un alkyle en C₁ à C₆ qui peut être linéaire ou ramifié, ou CH₂CO₂R^{VI} dans laquelle R^{VI} représente l'hydrogène ou un alkyle en C₁ à C₆ qui peut être linéaire ou ramifié

2. Composé chimique de la revendication 1, dans lequel R⁴ et R⁵ représentent l'hydrogène et R¹¹ représente le méthyle.

3. Composé chimique de la revendication 1, dans lequel R⁴ et R⁵ représentent l'hydrogène et R¹¹ représente l'éthyle.

4. Composé chimique de la revendication 1, dans lequel R⁴ représente l'hydrogène, R⁵ représente le diméthylsulfamoyle et R¹¹ représente le méthyle.

5. Composé chimique de la revendication 1, dans lequel R⁴ représente l'hydrogène, R⁵ représente le diméthylsulfamoyle et R¹¹ représente le méthyle.

6. Composition pharmaceutique utile pour le traitement d'un trouble d'un mammifère qui est sensible au blocage des récepteurs des acides glutamique et aspartique, comprenant une quantité du composé chimique selon l'une quelconque des revendications 1 à 5 qui est efficace pour un tel but, conjointement avec un excipient pharmaceutiquement acceptable.

7. Utilisation du composé chimique selon l'une quelconque des revendications 1 à 5, pour la fabrication d'un médicament utile dans le traitement des troubles d'un mammifère, y compris un homme, qui sont sensibles au blocage des récepteurs des acides glutamique et aspartique.

8. Utilisation d'un composé chimique selon l'une quelconque des revendications 1 à 5, pour la fabrication d'un médicament utile dans le traitement des troubles cérébrovasculaires ou psychotiques.

9. Procédé de préparation du composé chimique selon l'une quelconque des revendications 1 à 5, ce procédé comprenant l'étape consistant à faire réagir un composé représenté par la formule générale (II) 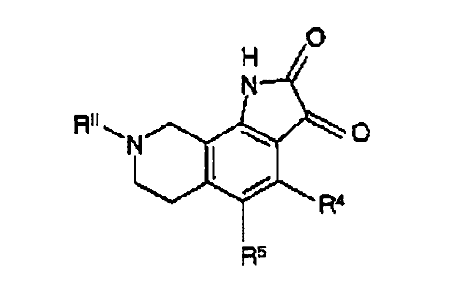 dans laquelle R⁴, R⁵ et R¹¹ sont tels que définis dans la revendication 1,
avec de l'hydroxylamine ou un dérivé réactif de celui-ci.
